# EUROPEAN PATENT SPECIFICATION

(11) **EP 3 720 731 B1**
(45) Date of publication and mention of the grant of the patent: **11.05.2022**
(21) Application number: 17835495.7
(22) Date of filing: 11.12.2017
(51) Int. Cl.: B60K 28/06, A61B 5/00, A61B 5/08, G01N 33/497

(54) **EQUIPMENT FOR MEASURING THE HUMAN ALCOHOL INTOXICATION LEVEL TO PREVENT FALSIFICATION OF MEASUREMENT AND RESULTS AND METHOD FOR MEASURING**
VORRICHTUNG ZUR MESSUNG DES MENSCHLICHEN ALKOHOLINTOXIDATIONSGRADES ZUR VERMEIDUNG EINER VERFÄLSCHUNG DER MESSUNG UND ERGEBNISSE SOWIE VERFAHREN ZUR MESSUNG
ÉQUIPEMENT POUR MESURER UN NIVEAU D'IMPRÉGNATION ALCOOLIQUE HUMAINE POUR EMPÊCHER LA FALSIFICATION D'UNE MESURE ET DE RÉSULTATS ET PROCÉDÉ POUR LA MESURE

(30) Priority: 04.12.2017 LT 2017538
(43) Date of publication of application: 14.10.2020
(73) Proprietor: UAB Universumas, 02184 Vilnius (LT)
(72) Inventor: GRIGALIUNAS, Rytis, LT-02184 Vilnius (LT)
(74) Representative: Zaboliene, Reda
(86) International application number: PCT/IB2017/057775
(87) International publication number: WO 2019/111044

(56) References cited:
- EP-A1- 2 075 151
- EP-A1- 2 237 034
- FR-A1- 3 042 280

## Description

### FIELD OF THE INVENTION

The invention relates to the field of alcohol intoxication measuring apparatuses, in particular to measuring apparatuses that minimize chances to cheat, falsify the measurement and results when the person who is testing for alcohol intoxication is not directly controlled by other persons concerned.

### DESCRIPTION OF THE RELATED ART

This invention provides a technical solution to help prevent fraud, falsification of measurement and results in estimating the human alcohol intoxication level when a person who is testing for alcohol intoxication is not directly controlled by other persons concerned. The technical solution presented below minimizes to minimum the possibility of fraud during the measurement when the measurement is captured by means of image capture, and the measuring apparatus is replaced by another object, visually similar to the measuring apparatus. The technical solution also minimizes to minimum the possibility of diverting the air into the measuring apparatus not from the measured human lungs during the measurement, i.e. for example from external compressed air means (e.g. air balloons, etc.)

Document US4738333A (published on 19 April, 1988) provides a sobriety testing system designed for drivers with additional functions identifying a specific driver and ensuring the correctness of the sobriety testing. For a given system, a different physical act according to which the system identifies a particular driver is assigned for each driver, i.e. before the start of the drive the driver must perform a specific physical act assigned to him, according to which the system will identify the driver. A method of preventing fraud while testing sobriety also relates to the specific physical act assigned to the driver. The provided system is not convenient because the physical act must be performed accurately, otherwise the system prohibits driving, in such case it is very probable to miss the physical act, it can be difficult to accurately remember the physical act if it has not been performed for a long time, and in the case of trauma it is impossible to repeat it. No solutions are provided to avoid cases when the non-driver blows into the alcohol intoxication measuring apparatus, i.e. the source of the blown air is changed.

Document LT2015 086 (published on 11 April, 2016) provides a human alcohol intoxication measuring apparatus that captures the face of a person who blows into the measuring apparatus and identifies the person according to this information. In the case of the described measuring apparatus, it is possible to falsify the measurement by feeding air through a blow tube, whose end is not captured by an image recording camera, i.e. the measurement does not ensure the integrity of the equipment and it is possible to measure the blown air of the other person than the tested person.

Document US8249311B2 (published on 21 August, 2012) provides a system when during the measurement of sobriety; a face of a measured person is captured by image capture means and is compared to the face in the database. In the case of this solution, no decision is made to avoid fraud by replacing the measuring apparatus.

Document EP2237034B1 (published on 24 July, 2013) provides an alcohol measuring device, where the signal transmitter emits a signal for a communication with the, the camera is used to capture the face of the person, using the device. However, there are some drawbacks: the person could attach the additional tube, connecting the alcohol measuring device with the smart device or computer. Such changes would not be detected.

Document EP2075151 A1 (published on 13 July, 2011) provides an alcohol measuring device and an alcohol detecting device. The appearance of a user is photographed, and it is determined if the alcohol measurement is properly carried out. However, there is no coded communication between the alcohol measuring device and the alcohol detecting device, so the alcohol measuring device could be replaced by other. In addition, the user could attach the additional tube, connecting the alcohol measuring device with the smart device or computer. In our case, the alcohol measuring device emits coded signals, which are detected by the alcohol detecting device. 3D identification of the alcohol measuring device is used in our invention, which eliminates the possibility to damage or change the alcohol measuring device.

Document FR3042280A1 (published on 27 December, 2019) provides a portable terminal connected to a sensor, measuring the alcohol concentration, and equipped with a camera to take an image of the individual. The sensor has the marker, a signal code, which allows to identify the senser, and it could not be replaced. The user is also identified. However, the sensor is identified only using a 2d identification. 3D identification of the alcohol measuring device is used in our invention, which eliminates the possibility to damage or change the alcohol measuring device, which could not be detected by the 2D identification. In addition, a light source for illumination a tube of the alcohol testing apparatus is used in our invention, thus eliminating the possibility to damage or attach another tube.

Summarizing documents of the current related art, the following deficiencies can be distinguished:
- the alcohol intoxication measurement system requires a very complex and long-lasting identification of the tested person;
- there are no solutions for avoiding the deceptive measurement of sobriety when a non-human blows into the measuring apparatus or other person than it is required for a specific situation (i.e., another person is asked to perform the measurement);
- no solutions are provided to ensure that the actual measuring apparatus is not replaced by another visually similar device.

This invention provides a technical solution that does not have the above mentioned deficiencies.

### SUMMARY OF THE INVENTION

This invention provides a new technical solution for determining the human alcohol intoxication level, where the said solution has technical features that protect against fraud and falsification. The technical solution includes the method for measuring and equipment that implements this method. The equipment according to claim 1 consists of at least two main devices - a smart device and an alcohol testing apparatus, where both of these devices have technical means for intercommunication. In the present invention, there are used two types of communication between the alcohol testing apparatus and the smart device: optical and for data transfer. The optical communication is intended to reduce the possibility of fraud by replacing the alcohol testing apparatus with another, similar object; data transfer communication is intended for data transfer between the alcohol testing apparatus and the smart device. Equipment and method for measuring allows to identify whether the person really touches a transparent luminous tube of the alcohol testing apparatus to identify whether there are no external or other air-feed devices in the tube or the integrity of the tube is intact.

The method for measuring the alcohol intoxication level according to claim 8 consists of at least the following main stages: before the measurement the tested person positions the smart device in the way that in the camera vision field of the device the 3D identification of the device will be performed, and then the face of the person will appear in the camera capture field of the device, necessarily the lips together with the alcohol testing apparatus; when measuring, the alcohol testing apparatus transfers the signal of the alcohol testing apparatus used, which confirms that the smart device captures (and at the same the tested person uses) the specific alcohol testing apparatus via optical communication.

As usual measurement data is transferred to the persons concerned by the smart device via data networks or other means for analysis, decisions, etc. Using this technical solution, you can measure alcohol intoxication of distant persons, and this solution ensures that during the measurement, the possibility of fraud, falsification of the measurement or its results is minimized.

### BRIEF DESCRIPTION OF THE DRAWINGS

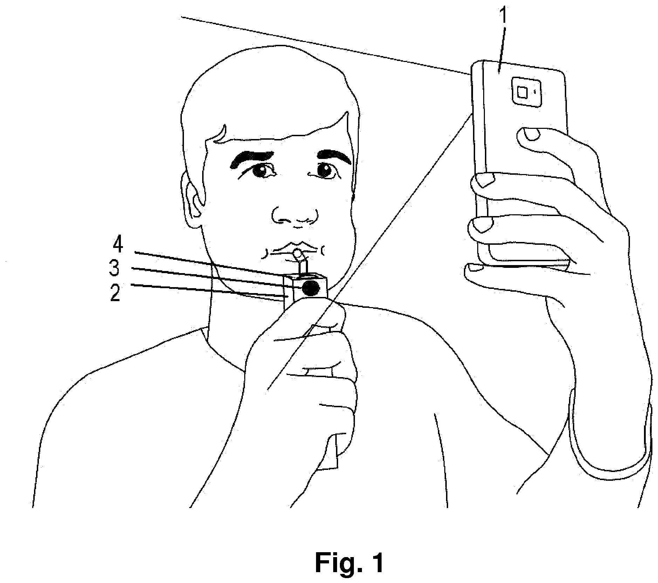

Figure 1 depicts a technical solution (an alcohol testing apparatus - a smart device) during the measurement of alcohol intoxication.

The given figure is more illustrative, scale, proportions and other aspects do not necessarily correspond to a real technical solution.

### THE PREFERRED EMBODIMENTS

One of the ways to determine the human alcohol intoxication level is to measure the amount of alcohol in human blood. The amount of alcohol in the blood can be determined by measuring the composition of the breath of the person. A wide range of widely known alcohol testing apparatuses are intended for this purpose. When measuring alcohol intoxication, cases of fraud occur when the tested person is not directly controlled by other persons concerned; in order to reduce the indication of the alcohol testing apparatus, for example: a person who blows into the alcohol testing apparatus is not the one whose alcohol intoxication level needs to be measured, compressed air systems are used, the flow formed by compressed air systems is directed to the measuring apparatus, the measuring apparatus is replaced by a different, visually similar device and other types of fraud. In order to prevent fraud, falsification, besides the tested person, the person who is responsible for the correct measurement is often involved, but the presence of the additional, responsible person complicates the measurement, greatly reduces the flexibility of measurement options, reduces the applicability in different situations, and does not allow testing and performing alcohol intoxication of distant persons. Another way to ensure the measurement without fraud is to develop a measuring apparatus specifically tailored to reduce the risk of fraud when measured. As outlined in the overview of the related art, one of the ways to avoid fraud is to capture a face of a measured person, but in this case the situation when the measuring apparatus is replaced by another device that is similar to the measuring apparatus may arise. This invention provides a technical solution that minimizes to minimum the possibility of replacing a device with a visually similar device, reduces other possibilities of fraud during the measurement of alcohol intoxication.

This invention provides a new technical solution for measuring alcohol intoxication with additional means to prevent fraud during the measurement. The technical solution consists of equipment containing at least these components:
- a smart device (1) with an image capture camera;
- a special alcohol testing apparatus (2), having communication with the smart device (1).

In the context of this invention, **a smart device (1)** is understood as a standard means of the related art. The smart device (1) is an electronic device with processor(s) for data processing, various memory modules, internal and external data communication modules, image capture and display modules, and other components specific to such devices. In the case of the present invention, it is convenient to use the smart device (1) that has an image capture camera and image display on the same side of the device, i.e. seeing himself on the screen a person can conveniently capture using the image capture camera. In other embodiments, the image capture camera and image display screen are on different sides of the device (1). As it is customary for smart devices, the device of the present invention (1) is also provided with software means for controlling the device (1). In the case of the present invention, the smart device (1) has special software means for controlling the alcohol intoxication measurement process, whose operation is described in this description. Also the smart device (1) has software means for capturing and/or recognizing the captured face of the tested person and assigning the data in the database, i.e. to associate the tested person with his data in the database. Also the smart device (1) has software means for analyzing an image captured by an illuminated translucent tube of the alcohol testing apparatus (2) to determine whether external sources of the measured air are used, i.e. to determine the integrity of the tube. Also, the smart device (1) has software means for measuring and analyzing the temperature of the human air flow. Also the smart device (1) has software means for measuring and analyzing the speed of human air flow and speed stability.

Another special device required for the implementation of the present invention is **an alcohol testing apparatus (2).** In the case of this invention, in addition to the usual components necessary for measuring alcohol intoxication, the alcohol testing apparatus (2) has technical means for communication between the alcohol testing apparatus (2) and the smart device (1). Technical means are used to ensure two types of communication: means for optical and means for data transfer communication. In the case of this alcohol testing apparatus (2), the tube through which the tested person has to blow the air to the alcohol testing apparatus (2) is fastened to the alcohol testing apparatus (2) in the way that during the measurement, the smart device (1) can capture the entire tube from lips of the tested person to a connection of the tube with the alcohol testing apparatus (2).

The alcohol testing apparatus (2) have technical means for measuring the temperature of the blown air, for example, a thermometer which changes the temperature to a corresponding electrical signal or signal parameters, whose analysis can determine the temperature of the blown air. Other means protecting against falsification: the alcohol testing apparatus (2) has means for measuring the speed of the blown air and speed stability during the measurement. If the temperature, speed, change in speed of the blown air during the measurement is significantly different from the set value, additional means are taken (e.g. to repeat the measurement, make an additional 3D identification of the alcohol testing apparatus, information on possible falsification is being transferred to the persons concerned) to avoid falsification.

Means for implementation of **optical communication** on the smart device (1) are means for image capture and analysis of data on captured images, and means for generation and control of optical communication parameters. Means for implementation of optical communication in the alcohol testing apparatus (2) include a light source (3) and its technical means for controlling. During the measurement of alcohol intoxication (either immediately before the measurement or immediately after the measurement), the aforementioned light source (3) blinks according to the order set by means for optical communication control, i.e. according to the identification signal given by means for control, durations of the illuminating (radiating, emitting) light source (3) and the not illuminating (not radiating, not emitting) source (3), other parameters. Every time when alcohol intoxication is measured, the light source (3) is blinking in an identifying sequence. In other embodiments, the light source (3) emits radiation whose optical spectrum is outside the human visible spectrum, but which can be captured by the image capture camera (1) of the smart device (for example, infrared rays). When measuring alcohol intoxication, the blinking light source (3) of the alcohol testing apparatus (2) must necessarily appear in the image capture field of the image capture camera of the smart device i.e. a direct optical, visual connection between the alcohol testing apparatus (2) and the smart device (1) must be. When the light source (3) forms a unique sequence, the said sequence is captured and analyzed by the smart device (1). The said light source (3) in the alcohol testing apparatus (2) is positioned in such place of the alcohol testing apparatus (2) that during the measurement it falls into the image capture field of the smart device (1).

Another additional light source (4) is placed in such place of the alcohol testing apparatus (2) in order to illuminate the face of the tested person. The luminaire of the face of the tested person is used to improve the illuminating conditions so that the smart device (1) can easily capture the face of the tested person. In other embodiments of the invention, the second, additional light source (4) may also blink according to the set order and, in combination with the light source described above, (3) form a sequence for identifying the alcohol testing apparatus (2).

Other additional light sources in the alcohol testing apparatus (2) can be positioned in such places that it is possible for the smart device (1) to ensure the identification of the alcohol testing apparatus (2) by rotating the camera around at other angles in front of the alcohol testing apparatus (2). In this way, the 3D identification of the alcohol testing apparatus is implemented, i.e. the smart device (1) captures the rotation of the alcohol testing apparatus (2) in front of the camera, analyzes the order and sequence of the blinking of light sources according to the rotation.

At the point where the tube of the alcohol testing apparatus (2) connects with the alcohol testing apparatus (2) the light source for illumination of the tube is placed. In the case of this invention, a transparent, translucent tube is used. The light source for illumination of the tube illuminate to the edge of walls of the tube of the alcohol testing apparatus (2). The light emitted by the light source runs through the walls of the tube. In this way, in order to falsify the measurement by violating the tube of the alcohol testing apparatus (2), the light propagation through walls of the tube would be violated too. Such violation is captured by the image capture camera of the smart device (1).

In one embodiment of the present invention, the said light source for illumination of the blinks according to a set sequence, order as described above. Optical communication can both identify the alcohol testing apparatus (2) (confirm that a suitable alcohol testing apparatus is used) and ensure the integrity of the tube of the alcohol testing apparatus (2) (to ensure that external, additional air sources are not used to direct the air to the alcohol testing apparatus (2)).

Another type of communication between the smart device (1) and the alcohol testing apparatus (2) is used to **transfer data between themselves.** From a physical point of view, the said communication for transfer of data is realized using electromagnetic radiation, which can be used for data transfer by modulating, modifying in various ways. In the context of this invention, smart devices use conventional wireless data transfer methods such as Bluetooth, Wi-Fi, NFC, IrDA, or others. In other embodiments of the invention, instead of said wireless transfer methods, transfer methods using wires, cables, fiber optics, or other material data transfer channels may be used. For the said communication between the smart device (1) and the alcohol testing apparatus (2) to transfer data, technical means of standard techniques such as electromagnetic radiation generators and receivers, radiation modulators, antennas, devices for transfer and reception of data, and others are used.

A method for measuring the human alcohol intoxication level, including at least the following measurement steps:
- the alcohol testing apparatus (2) is activated, special software means are activated on the smart device (1);
- the tested person holds the smart device (1) in the hand or positions in such place that the capture field of the smart device (1) captures the entire face of the tested person necessarily the lower part of the face, where it is clearly possible to capture human lips and a clear tube of the alcohol testing apparatus (2) to which it is necessary to blow up the breath;
- the tested person positions the alcohol testing apparatus (2) in such way that the light source (3) is placed in the capture field of the image capture camera of the smart device (1) and blown into the alcohol testing apparatus (2);
- during the measurement, the alcohol testing apparatus (2) forms the identifying sequence for the pulsed light source (3), and other optical communication light sources, the light sources are blinking the sequence identifying the alcohol testing apparatus (2), at the same time the image camera of the smart device (1) captures the tested person and the said sequence of the light source (3).

The order/sequence of blinking of the light source (3) of the alcohol testing apparatus (2) is captured by the image capture camera of the smart device (1), the sequence is compared to sequences in the smart device (1) to make sure that the alcohol testing apparatus (2) is suitable (2), the alcohol testing apparatus (2) has not been replaced by a different, visually similar device. The light source (3)/ sources (3) and (4) of the alcohol testing apparatus (2) forms an optical pulse sequence whose parameters are matched to the smart device (1).

One of the possible ways of forming a sequence of blinking of light sources (3), (4) of the alcohol testing apparatus (2) is when a set of sequences is formed. The said set of sequences is installed in software means of the smart device (1), and the other, identical to the set of the smart device(1) is installed in the alcohol testing apparatus (2), i.e. the alcohol testing apparatus (2) and smart device (1) have an identical set of sequences. During the measurement, the smart device (1) reports to the alcohol testing apparatus (2) which sequence the alcohol testing apparatus (2) should generate and display, and the smart device (1) captures the display sequence and compares it with the sequence recorded in software means of the smart device (1). If the sequence matches, the identification of the alcohol testing apparatus (2) is correct. Other principles of formation, generation and transfer of sequences are possible too. The most important property of the present invention is that light sources (3) of the alcohol testing apparatus (2) and / or other described light sources of the alcohol testing apparatus (2) must be in the image capture field/coverage of the smart device (1) and the smart device (1) identifies the alcohol testing apparatus (2) according to the order of blinking of light sources.

The flow measurement data is transferred from the alcohol testing apparatus (2) to the smart device (1) via the data transfer communication. The smart device (1) analyzes the captured image, confirms that the suitable alcohol testing apparatus (2) is used, and transfer the collected and aggregated information for further processing, use, and decision making.

To illustrate and describe this invention, a description of preferred embodiments is given above. This is not a complete or limiting description aimed at prescribing a precise form or embodiment. The description given above should be regarded more as an illustration than a constraint. Obviously, the specialists in this field may clearly see a multitude of modifications and variations. The embodiment has been selected and described so as to enable the specialists in this field to best understand the principles behind this invention and their best practical application for different embodiments with different modifications fit for a specific application or embodiment customization. The invention scope is defined by the attached claims.

Embodiments described by specialists in the respective field may contain changes that do not depart from the scope of this invention, as described in the claims given next.

## Claims

1. An equipment for measuring the human alcohol intoxication level including:
a smart device (1) with an image capture camera;
a special alcohol testing apparatus (2) configured to communicate with the smart device (1);
wherein the special alcohol testing apparatus (2) comprises:
- at least one light source (3) for optical communication with the smart device (1);
**characterized in that** the special alcohol testing apparatus (2) further comprises:
- a light source arranged to illuminate a tube of the alcohol testing apparatus (2), the light source being fastened
in a place where the tube connects with the alcohol testing apparatus (2), and said light source being arranged to illuminate to the edge of walls of the tube of the alcohol testing apparatus (2);
and the smart device (1) comprises technical and software means configured for 3D identification of the alcohol testing apparatus (2), when the alcohol testing apparatus (2) is rotated at different angles with respect to the camera.

2. The equipment for measuring the human alcohol intoxication level according to the preceding claims, **characterized in that** it has a light source (4) for illumination of a face of the tested person during the measurement.

3. The equipment for measuring the human alcohol intoxication level according to the preceding claims, **characterized in that** light sources of the alcohol testing apparatus (2) generate an optical pulse sequence whose parameters are identified by the smart device (2).

4. The equipment for measuring the human alcohol intoxication level according to the preceding claims, **characterized in that** light sources of the alcohol testing apparatus (2) emit radiation that is outside the spectrum of the human visible spectrum, but that can be captured by an image capture camera of the smart device (1)

5. The equipment for measuring the human alcohol intoxication level according to the preceding claims, **characterized in that** the smart device (1) has technical means for capturing a blinking sequence of light sources of the alcohol testing apparatus (2).

6. The equipment for measuring the human alcohol intoxication level according to the preceding claims, **characterized in that** the alcohol testing apparatus (2) has technical means for measuring the temperature of a blown air, for example, a thermometer which changes the temperature to a corresponding electrical signal or signal parameters, which can be determined by analyzing the temperature of the blown air.

7. The equipment for measuring the human alcohol intoxication level according to the preceding claims, **characterized in that** the alcohol testing apparatus (2) has technical means for measuring the speed of the blown air and the change in the speed during measurement.

8. A method for measuring the human alcohol intoxication level, including at least the following measurement stages:
- an alcohol testing apparatus (2) is activated, special software means are activated on a smart device (1);
- a tested person holds the smart device (1) in a hand or positions such that a capture field of the smart device (1) captures an entire face of the tested person, necessarily a the lower part of the face, where it is clearly possible to capture human lips and a clear tube of the alcohol testing apparatus (2) to which it is necessary to blow up the breath;
- the tested person positions the alcohol testing apparatus (2) in such a way that a light source (3) that illuminates the alcohol testing apparatus (2) is placed in a capture field of an image capture camera of the smart device (1),
wherein during the measurement:
- the alcohol testing apparatus (2) forms an identifying sequence for the pulsed light source (3), and other optical communication light sources, which are determined and matched with the smart device (1), the light sources are blinking the sequence identifying the alcohol testing apparatus (2), and at the same time the image camera of the smart device (1) captures the tested person and said sequence of the light source;
- the alcohol testing apparatus (2) is recognized using a 3D identification;
- the light source illuminates an edge of walls of the tube of the alcohol testing apparatus (2) and identifies whether there are no external or other air-feed devices in the tube or the integrity of the tube is intact;
- the temperature of the human blown air is measured, the measured values are compared with set values;
- the speed of the human blown air and change in speed is measured and the measured values are compared with the set values;
- if the temperature and the speed of the human blown air values are different from the set values, additional means are initiated to properly identify the tested person and the alcohol testing apparatus (2).

9. The method according to claim 8, wherein technical means according to claims
1-7 are used for implementation.

## Patentansprüche

1. Eine Anlage zum Messen des menschlichen Alkoholvergiftungsniveau einschließlich
eines intelligenten Gerätes (1) mit einer Bilderfassungskamera;
eines speziellen Alkoholtestapparat (2), der auf Kommunikation mit dem intelligenten Gerät (1) eingerichtet ist;
wobei der Alkoholtestapparat (2) aus dem folgenden besteht:
- zumindest eine Lichtquelle (3) für optische Kommunikation mit dem intelligenten Gerät (1); ausgezeichnet dadurch, dass der spezielle Alkoholtestapparat (2) darüber hinaus beinhaltet:
- eine Lichtquelle, die dazu eingerichtet ist, einen Schlauch des Alkoholtestapparates (2) zu beleuchten.
Die Lichtquelle ist befestigt an einer Stelle, an der der Schlauch mit dem Alkoholtestapparat (2) verbunden ist
und die erwähnte Lichtquelle ist darauf angelegt in die Kanten der Schlauchwände des Alkoholtestapparates (2) zu beleuchten;
und das intelligente Gerät (1) enthält technische und Computer-Software-Mittel die für die 3D-Identifitzierung des Alkoholtestapparates (2) eingerichtet sind, wenn der Alkoholtestapparat (2) in einen anderen Winkel im Bezug zur Kamera rotiert wird.

2. Die Ausrüstung zum Messen des menschlichen Alkoholvergiftungsniveaus entsprechend der oben genannten Anforderungen, die sich durch die Lichtquelle (4) für die Beleuchtung des Gesichtes der Testperson während der Messung ausgezeichnet.

3. Die Ausrüstung zum Messen des menschlichen Alkoholvergiftungsniveaus entsprechend der oben genannten Anforderungen, die sich dadurch auszeichnet, dass die Lichtquelle des Alkoholtestapparates (2) eine optische Impulssequenz erzeugt, derer Parameter von dem intelligenten Gerät (2) identifiziert werden

4. Die Ausrüstung zum Messen des menschlichen Alkoholvergiftungsniveaus entsprechend der oben genannten Anforderungen, die sich dadurch auszeichnet, dass die Lichtquellen des Alkoholtestapparats (2) Strahlen emittieren, die sich außerhalb des menschlich sichtbaren Spektrums befinden, aber von der Bilderfassungskamera des intelligenten Geräts (1) erfasst werden können.

5. Die Ausrüstung zum Messen des menschlichen Alkoholvergiftungsniveaus entsprechend der oben genannten Anforderungen, die sich dadurch auszeichnet, dass das intelligente Gerät (1) über die technischen Fähigkeiten verfügt eine Blinksequenz der Lichtquelle des Alkoholtestapparates (2) zu erfassen.

6. Die Ausrüstung zum Messen des menschlichen Alkoholvergiftungsniveaus entsprechend der oben genannten Anforderungen, die sich dadurch auszeichnet, dass der Alkoholtestapparat (2) über die technische Fähigkeit zur Temperaturmessung der geblasenen Luft verfügt, zum Beispiel mit einem Thermometer, welches die Temperatur mit einem korrespondierenden elektrischen Signal oder Signalparametern austauscht, die mittels Analyse der geblasenen Lufttemperatur bestimmt werden können.

7. Die Ausrüstung zum Messen des menschlichen Alkoholvergiftungsniveaus entsprechend der oben genannten Anforderungen, die sich dadurch auszeichnet, dass der Alkohohltestapparat (2) über die technischen Funktionen verfügt, um die Geschwindigkeit der geblasenen Lust und den Geschwindigkeitswechsel während der Messung zu messen.

8. Die Ausrüstung zum Messen des menschlichen Alkoholvergiftungsniveaus, die zumindest alle der folgenden Messstadien einschließt:
- ein Alkoholtestapparat (2), der aktiviert ist, spezielle Software-funktionen sind aktiv auf dem intelligentem Gerät (1);
- eine Testperson hält das intelligente Gerät (1) in einer Hand oder in Positionen, die in das Erfassungsfeld des intelligentem Geräts (1) fallen, und erfasst das gesamte Gesicht der Testperson, notwendigerweise
den unteren Gesichtsbereich, in dem es deutlich möglich ist, die menschlichen Lippen und einen durchsichtigen Schlauch des Alkoholtestapparates (2), in Atemluft geblasen werden muss, zu erfassen;
entsprechend der oben genannten Anforderungen,
- Die Testperson positioniert den Alkoholtestapparat (2) auf die Weise in die Lichtquelle (3), welche den Alkoholtestapparat (2) erleuchtet und im Erfassungsfeld der Bilderfassungskamera (1) platziert wird, wodurch während der Messung:
- der Alkoholtestapparat (2) eine identifizierende Sequenz für die pulsierende Lichtquelle (3) bildet und andere optische Lichtquellen, welche bestimmt und abgestimmt mit dem intelligentem Gerät (1), die Lichtquellen die Sequenz zur Identifikation an den Alkoholtestapparat (2) blinken und gleichzeitig die Bildkamera des intelligenten Geräts (1) die Testperson und die erwähnte Lichtquellensequenz erfasst;
- der Alkoholtestapparat (2) wird unter Nutzung einer 3D-Identifizierung erkannt;
- Die Lichtquelle erleuchtet die Enden der Schlauchwände der Alkoholtestapparates (2) und identifiziert, ob es keine externen oder anderen luftgespeisten Geräte im Schlauch gibt oder ob die Integrität des Schlauches intakt ist;
- Die Temperatur der menschlichen Blasluft wird gemessen, die gemessenen Werte werden mit den eingestellten Werten verglichen;
- Die Geschwindigkeit der menschlichen Blasluft wird gemessen, die gemessenen Werte werden mit den eingestellten Werten verglichen;
- wenn die Temperatur und die Geschwindigkeit der menschlichen Blasluft sich von den eingestellten Werten unterscheidet, werden zusätzliche Maßnahmen zur entsprechenden Identifizierung der Testperson und des Alkoholtestapparates (2) eingeleitet.

9. Eine Methode gemäß Anspruch 8, wobei die technischen Fähigkeiten der Ansprüche 1-7 für die Umsetzung genutzt werden.

## Revendications

1. Un équipement pour mesurer le niveau d'intoxication humaine à l'alcool comprenant :
un dispositif intelligent (1) avec une caméra de capture d'image ;
un appareil de test d'alcoolémie spécial (2) configuré pour communiquer avec le dispositif intelligent (1) ;
dans lequel l'appareil de test d'alcoolémie spécial (2) comprend :
- au moins une source de lumière (3) pour une communication optique avec le dispositif intelligent (1) ; **caractérisé en ce que** l'appareil de test d'alcoolémie spécial (2) comprend en outre
- une source de lumière agencée pour éclairer un tube de l'appareil de test d'alcoolémie (2),
la source lumineuse étant fixée à un endroit où le tube se raccorde à l'appareil de test d'alcoolémie (2),
et ladite source de lumière étant agencée pour éclairer jusqu'au bord des parois du tube de l'appareil de test d'alcoolémie (2) ;
et le dispositif intelligent (1) comprend des moyens techniques et logiciels configurés pour une identification 3D de l'appareil de test d'alcoolémie (2), lorsque l'appareil de test d'alcoolémie (2) est tourné sur différents angles par rapport à la caméra.

2. L'équipement de mesure du taux d'intoxication humaine à l'alcool selon les revendications précédentes, **caractérisé en ce qu'**il comporte une source lumineuse (4) pour éclairer le visage de la personne testée pendant la mesure.

3. L'équipement de mesure du taux d'intoxication humaine à l'alcool selon les revendications précédentes, **caractérisé en ce que** les sources lumineuses de l'appareil de test d'alcoolémie (2) génèrent une séquence d'impulsions optiques dont les paramètres sont identifiés par le dispositif intelligent (2).

4. L'équipement de mesure du taux d'intoxication humaine à l'alcool selon les revendications précédentes, **caractérisé en ce que** les sources lumineuses de l'appareil de test d'alcoolémie (2) émettent un rayonnement qui est en dehors du spectre du visible humain, mais qui peut être capté par la caméra de capture d'image du dispositif intelligent (1).

5. L'équipement de mesure du taux d'intoxication humaine à l'alcool selon les revendications précédentes, **caractérisé en ce que** le dispositif intelligent (1) comporte des moyens techniques pour capturer une séquence de clignotement des sources lumineuses de l'appareil de test d'alcoolémie (2).

6. L' équipement de mesure du taux d'intoxication humaine à l'alcool selon les revendications précédentes, **caractérisé en ce que** l'appareil de test d'alcoolémie (2) comporte des moyens techniques pour mesurer la température d'un air soufflé, par exemple, un thermomètre qui transforme la température en un signal électrique correspondant ou en paramètres de signal, qui peuvent être déterminés en analysant la température de l'air soufflé.

7. L'équipement de mesure du taux d'intoxication humaine à l'alcool selon les revendications précédentes, **caractérisé en ce que** l'appareil de test d'alcoolémie (2) comporte des moyens techniques pour mesurer la vitesse de l'air soufflé et la variation de la vitesse pendant la mesure.

8. Un procédé de mesure du niveau d'intoxication humaine à l'alcool, comprenant au moins les étapes de mesure suivantes :
- un appareil de test d'alcoolémie (2) est activé, des moyens logiciels spéciaux sont activés sur un dispositif intelligent (1) ;
- une personne testée tient le dispositif intelligent (1) dans une main ou des positions de sorte que le champ de capture du dispositif intelligent (1) capture le visage entier de la personne testée, nécessairement la
partie inférieure du visage, où il est clairement possible de saisir des lèvres humaines et un tube transparent de l'appareil de contrôle de l'alcoolémie (2) auquel il est nécessaire de souffler l'haleine ;
- la personne testée positionne l'appareil de test d'alcoolémie (2) de telle sorte qu'une source de lumière (3) qui éclaire l'appareil de test d'alcoolémie (2) est placée dans le champ de capture d'une caméra de capture d'image du dispositif intelligent (1),
où, pendant la mesure :
- l'appareil de test d'alcoolémie (2) forme une séquence d'identification pour la source de lumière pulsée (3),
et d'autres sources lumineuses de communication optique, qui sont déterminées et
appariées avec le dispositif intelligent (1), les sources lumineuses clignotent la séquence identifiant l'appareil de test d'alcoolémie (2), et en même temps la caméra d'image de l'appareil du dispositif intelligent (1) capture la personne testée et ladite séquence de la source lumineuse ;
- l'appareil de test d'alcoolémie (2) est reconnu à l'aide d'une identification 3D ;
- la source lumineuse éclaire un bord des parois du tube de l'appareil de test d'alcoolémie (2) et identifie s'il n'y a pas de dispositif d'alimentation en air externe ou autre dans le tube ou si l'intégrité du tube est intacte ;
- la température de l'air soufflé par l'homme est mesurée, les valeurs mesurées sont comparées aux valeurs définies ;
- la vitesse de l'air soufflé par l'homme et le changement de vitesse sont mesurés et les valeurs mesurées sont comparées aux valeurs définies ;
- si les valeurs de température et de vitesse de l'air soufflé par l'être humain sont différentes des valeurs définies, des moyens supplémentaires sont mis en oeuvre pour identifier correctement la personne testée et l'appareil de test d'alcoolémie (2).

9. Le procédé selon la revendication 8, dans laquelle des moyens techniques selon les revendications 1 à 7 sont utilisés pour la mise en oeuvre.
